# EUROPEAN PATENT APPLICATION

(11) **EP 1 433 387 A1**
(43) Date of publication of application: **30.06.2004**
(21) Application number: 02356270.5
(22) Date of filing: 26.12.2002
(51) Int. Cl.: A23L 1/275, A23L 1/30, A23L 1/0562, A23P 1/02, A61K 9/16

(54) **Solid granules containing carotenoids**

(71) Applicant: Adisseo France S.A.S., 92160 Antony (FR)
(72) Inventor: Chiavazza, Véronique, 69300 Caluire (FR); Dollat, Jean-Marie, 03100 Montlucon (FR); Fayard, Sylvie, 69100 Villeurbanne (FR)
(74) Representative: Guerre, Dominique

(57) **Abstract**

The solid granules of the invention comprise at least 5% (w/w) of carotenoid, gelatine and a sugar, they have a size distribution of from 100 µm to 2000 µm and are solvent-free.

The process for producing such granules comprises the following steps:
(a) preparing an aqueous solution of at least gelatine and a sugar,
(b) adding at least said carotenoid in said aqueous solution,
(c) adding the preparation obtained in (b) to an oil to obtain an emulsion of granules,
(d) cooling said emulsion to solidify the granules, and
(e) recovering and drying the granules.

## Description

The present invention relates to free-flowing, stable and high-content-carotenoid granules and a process for producing such granules containing more than 5% of carotenoid, particularly lutein pigment.

Carotenoid pigment compositions are known, particularly spray-dried carotenoid compositions.

In the JP patent 1973-124124 water dispersible carotenoid are prepared by drying emulsions of the oil in a mixture of arabic gum and dextrin. But later, most of the compositions, for example described in the EP-A-278 284, contain gelatin, which is a very successful gelling agent used in compositions and conserves the stability with regard to light, temperature and oxidation. But these compositions contain generally a low level of pigment, not more than 5 % w/w.

A recent patent US-A1-2002/0052421 describes the preparation of carotenoid beads with higher concentrations but no information about their stability is given. And the process used to make granules is still spray-drying.

For carotenoid pigments very sensitive to heat, the spray-drying process presents the disadvantage to use a temperature quite high even it is applied during a short time. Furthermore the granules obtained by spray-drying have a broad range of particle size with a lot of small particles. So the flowability is not so good.

EP-A-618 001 describes a process for producing granules comprising an active compound, in particular the vitamin A or E, but also carotenoids. In accordance with this process, higher amounts of the active compounds may be obtained, but this process uses a hydrocarbon solvent, in particular an aliphatic hydrocarbon having 6 carbon atoms, as the isohexane. The resulting granules are suitable for feeding broilers. Because of the high level of the dilution carried out on the vitamin granules in the feed, no significant amount of hydrocarbon solvent is detected.

On the contrary, such compositions can't be used for human consumption, because the granules are used either as such, or with a low level of dilution. The remaining solvent, even if present in very low amount, may have a harmful effect. Furthermore, in accordance with the process disclosed in EP-618 001, at least one surfactant should be used in the solvent. This burdens the recirculating process of the solvent because the surfactant should be first eliminated.

The present invention provides stable solid granules comprising a high content of at least one carotenoid and which are suitable for human consumption, in particular for a pharmaceutical, neutraceutical or nutritional supplementation use. In accordance with the invention, the granules comprise at least 5% carotenoid and are free from solvent, specifically hydrocarbon solvent.

Accordingly, the invention relates to solid solvent-free granules comprising at least 5% (w/w) carotenoid, further comprising gelatine and a sugar, and having a size distribution of from 100 µm to 2000 µm.

The present invention also provides a process for obtaining the above-defined carotenoid granules wherein no toxic solvent is used. Said process comprises the following steps:
(a) preparing an aqueous solution of at least gelatine and a sugar,
(b) adding at least said carotenoid in said aqueous solution,
(c) adding the preparation obtained in (b) to an oil to obtain an emulsion of granules,
(d) cooling said emulsion to solidify the granules, and
(e) recovering and drying the granules.

Preferably the oil of step (c) is of vegetable origin.

This process produces spherical granules which may be totally free from any solvent and surfactant, and therefore make them suitable for a human use.

Furthermore, the spherical granules thus obtained are steady and have a very good flowability.

The carotenoid is advantageously selected from the group consisting of lutein, zeaxanthin and their mixtures, said lutein or zeaxanthin being in their free form (hydroxide form) and/or their esterified form. The esterified form is generally a fatty esterified form, but the present invention encompasses any other esterified form of lutein or zeaxanthin. In particular, said carotenoid may be selected from oleoresin comprising at least esterified lutein and esterified zeaxanthin, saponified oleoresin comprising at least free lutein and free zeaxanthin, purified crystallized lutein obtained from natural source of carotenoids, and chemically prepared lutein or zeaxanthin. In accordance with the known methods for obtaining crystallized lutein from plant, the resulting lutein is obtained with a low amount of zeaxanthine; typically, the lutein amounts at least 90% (w/w) and the zeaxanthin amounts up to 6% (w/w).

The oleoresin may be extracted from any natural source of lutein, for example from Marigold flowers, but also from fruits as tomatoes, oranges, peaches, papayas, prunes and mangos. The oleoresin is generally extracted from a meal resulting of the drying and milling of corollas of Marigold flowers. The title of pigment (lutein + zeaxanthine) in the oleoresin is between 10 to 45% carotenoid depending on the extraction and/or saponification process.

The oleoresin may be present in an amount of from 10 to 40% (w/w), preferably from 20 to 30% (w/w).

Preferably, the granules comprise oleoresin containing at least 30% of carotenoid esters or purified or pure crystallized carotenoid. So the pigment or equivalent pigment (if supplied by an oleoresin) may be present in the granules in an amount of from 5 to 30% (w/w), preferably from 10 to 20% (w/w).

The granules of the present invention comprise gelatine, which may be present in the composition in an amount of from 15 to 50% (w/w), preferably from 30 to 45% (w/w). Any gelatine may be used in accordance with the present invention.

The granules further comprise a sugar. It may be selected from the group consisting of polyols, monosaccharides, disaccharides, glucose syrups and maltodextrines. The preferred polyols are selected from the group consisting of glycerol, sorbitol, maltitol and xylitol; the preferred monosaccharides are selected from the group consisting of fructose and glucose; and the preferred disaccharides are selected from the group consisting of lactose, maltose and sucrose. The amount of sugar advantageously varies from 10 to 50% (w/w), preferably from 20 to 35% (w/w) in the granules. When glucose syrup or maltodextrine is used, it is preferred that it has a Dextrose Equivalent (DE) of at least 25. The preferred sugar syrup is glucose syrup with a DE of between 45 and 65.

The granules of the invention may further comprise at least a fatty material obtained from an animal or vegetal source; suitably it has a vegetable origin. This materiel is preferably selected from the group consisting of fatty acids, fatty esters, derivatives thereof, for example triglyceride esters, and waxes. Preferably, the fatty material is solid at room temperature and liquid below 100°C. If the carotenoid is supplied with an oleoresin, the fatty material is further preferably miscible with said oleoresin. Suitably, the fatty acid has from 14 to 22 carbon atoms. Preferably, the fatty acid is stearic acid or a mixture of palmitic and stearic acids. Preferably the triglyceride ester is precirol.

The fatty material may be present in the granules in an amount up to 20% (w/w), preferably from 5 to 15% (w/w).

Said fatty material contributes to the stability of the granules, because at the temperature at which the process is carried out said fatty acid is liquid, and on cooling, it solidifies and remains solid at the temperature at which the granules are generally stored.

The granules may further comprises at least one antioxidant. A preferred antioxidant is selected from the group consisting of rosemary extracts, wine polyphenols extracts, ascorbic acid, sodium ascorbate, ascorbyl palmitate, tocopherols, derivatives of tocopherols, vitamin C, 3-tertiary butyl-4-hydroxyanisole (BHA), 3,5-di-tertiary-4-hydroxytoluene (BHT), 6-ethoxy-1,2-dihydroxy-2,2,4-trimethylquinoline (ethoxyquine). Rosemary extracts are more preferred. The antioxidant advantageously amounts up to 10% (w/w).

In some cases, it may be suitable to include an anti-caking agent in the granules. Compounds suitable for use as an anti-caking agent include silica magnesium stearate or starch. Preferably, the anti-caking agent agent is silica.

The anti-caking agent may be present in an amount of from 0 to 2 % (w/w), preferably from 0.2 to 1 % (w/w).

The granules may also comprise a finite amount of water. Suitably, the water is present in an amount of less than 10% (w/w).

The granules of the invention may be prepared by a method which involves the preparation of emulsions and is hereinafter referred to as the "double emulsion method". This method is advantageous because it forms spherical granules which have a good flowability. This process is another subject of the present invention.

Thus according to another aspect of the present invention there is provided a process for producing solid granules of carotenoid, as defined above, which comprises the following steps:
(a) a first step of preparing an aqueous solution of at least gelatine and a sugar;
(b) a second step of adding at least said carotenoid in said aqueous solution of step (a);
(c) a third step of adding the preparation obtained in (b) to an oil, to obtain an emulsion of granules;
(d) a fourth step of cooling said emulsion to solidify the granules; and
(e) a fifth step of recovering and drying the granules.

In accordance with the process of the invention, and in addition to the fact that no toxic solvent is used, no surfactant is needed. This renders the resulting granules typically intended for human consumption.

Advantageously, the oil of step (c) is a vegetable oil and is selected from the group consisting of rapeseed oil, corn oil, sunflower oil, soybean oil, palm oil, their mixtures and any ester thereof. Suitably, the oil is methyl esters of rapeseed oil.

Whereas no surfactant is needed, the oil may be recovered then directly recirculated.

The resulting granules may be used in the preparation of a vitamin mix suitable for use for food, cosmetic, nutraceutical or pharmaceutical applications. Thus the invention also relates to a food, cosmetic, nutraceutical or pharmaceutical composition comprising granules as defined above.

The present invention will now be described in more detail with reference to the following examples:

### General Method of Preparation

The particulate composition of the present invention was prepared using the following preferred process:
Step (1): In a first reactor, the sugar is dissolved in water at a preferred temperature from 50 to 70°C, ideally 60°C. The gelatine was added and mixed with stirring at a speed of 2 to 3 metres per second for at least twenty minutes whilst maintaining the temperature at 60°C.
Step (2): In a second reactor, in the case the pigment is supplied by a oleoresin, it is mixed with the melt fatty material used as diluant, - a preferred fatty material is miscible to the oleoresin - and with the antioxidant for 10 minutes to provide an oily liquid. To this effect, the antioxidant is preferably miscible with the oil. If the pigment is pure crystallized carotenoid, the addition of a fatty material may be applied or not; if this step is not carried out, no other prior preparation is required. But in this case, an antioxidant is advantageously added.
Step (3): The oily liquid obtained in step (2) was then added, with stirring, to the aqueous suspension prepared in step (1). Stirring was continued for 10 minutes whilst maintaining a temperature of 60°C to obtain
   - an oil in water emulsion if the raw material is an oleoresin
   - a good solid dispersion if the raw material is pure crystallized lutein.
Step (4): The emulsion or dispersion obtained in step (3) is then added to a liquid vegetable oil to obtain an (oil/water)/oil emulsion or water/oil emulsion.
Step (5): The temperature of the mixture is then reduced below 20°C, suitably 15°C (below the glass transition of the gelatine) to solidify the droplets of the oily phase. A gelatine-crosslinking agent is advantageously added in the oily mixture before the granules are recovered. Said agent may be a solution of glutaraldehyde.

The cooled mixture containing wet granules is then filtered or centrifuged.

The resulting particles are then dried in a fluidised bed at low temperatures, that means a granule temperature below 60°C.

### Example 1:

Granules of lutein esters are prepared as detailed above using the components given in Table 1 below:

| COMPONENT | CONCENTRATION of wet granules (%) | CONCENTRATION of dry granules (%) | WEIGHT (grams) |
|---|---|---|---|
| Gelatine 200 bloom | 16.25 | 30.2 | 195 |
| Lactose | 10.83 | 20.2 | 130 |
| Water | 50.00 | 6.0 . | 600 |
| Mari'Brite oleoresine With 39% lutein esters | 16.67 | 31.0 | 200 |
| Stearic acid | 6.25 | 11.6 | 75 |
| Silica | | 1.0 | |
| TOTAL | 100 | 100 | 1200 |

The lactose is dissolved in warm water (60C°). The gelatine is added to the lactose solution, stirring at a speed of 2 metres per second with a high-shear impeller.

The oleoresin is mixed with melt stearic acid at about 70°C and added under stirring to the aqueous phase. So a first emulsion oil in water is obtained.

This first emulsion is poured under stirring (helix impeller at 2.8 m/sec of peripherical speed) into 1.5 liter of a vegetable oil, here a rapeseed oil. A second emulsion of (oil/water) in rapeseed oil is obtained.

The droplets of aqueous phase are solidified by cooling the reactor at 15°C.

Then, 35 g of an aqueous solution of 16.7% glutaraldehyde is added in the rapeseed oil. After 20 minutes, the granules are filtrated, mixed with silica and dried one hour at room temperature and one hour at 60°C (air inlet temperature).

The particle size of the granules obtained ranged from 160 to 1000 microns with 50% in the range from 160 to 630 microns.

Because the initial oleoresin contains 39% of lutein esters, the theoretical amount of lutein and zeaxanthin, in the granules was calculated to be 12.1 % of lutein and zeaxanthin esters. The amount of lutein and zeaxanthin in granules measured one week after manufacturing is 11.8% that gives a 97.5% yield.

The amount of lutein and zeaxanthin, determined after four weeks of storage at 40°C in a dry atmosphere, is 11.3% equating to 96% stability.

### Example 2:

The procedure of Example 1 was repeated with the same amounts but the oil used in step (4) of the preparation method was methyl ester of rapeseed oil. Because of the lower viscosity of this oil, the granules have a smaller distribution size. The particle size range of the granules ranged from 100 to 1000 µm with 50% of particles from 160 to 500 µm.

The stability results are the same as example 1.

### Example 3:

The procedure of Example 1 was repeated with a different batch of oleoresin, a different kind of gelatine and quite different amounts of other products.

| COMPONENT | **CONCENTRATION** of wet granules (%) | **CONCENTRATION** of dry granules (%) | **WEIGHT** (grams) |
|---|---|---|---|
| Gelatine 140 bloom | 18.31 | 34.3 | 225 |
| Lactose | 12.21 | 22.9 | 150 |
| Water | 48.82 | 3.5 | 600 |
| Mari'Brite oleoresine With 43 % lutein esters | 15.78 | 29.6 | 195 |
| Stearic acid | 4.88 | 9.2 | 60 |
| Silica | | 0.5 | |
| TOTAL | 100 | 100 | 1230 |

After formation of the droplets. Then, 40 g of an aqueous solution of 16.7% glutaraldehyde is added in the methyl ester of rapeseed oil. After 20 minutes, the granules are filtrated, mixed with silica and dried one hour at room temperature and one hour at 60°C (air inlet temperature).

The particle size of the granules obtained ranged from 100 to 1000 microns with 45% in the range from 160 to 630 microns.

Because the initial oleoresin contains 43% of lutein esters, the theoretical amount of lutein and zeaxanthin in the granules was calculated to be 12.7 % of lutein and zeaxanthin esters. The amount of lutein and zeaxanthin in granules measured one week after manufacturing is 13 % that gives a 100 % yield.

The amount of lutein and zeaxanthin, determined after four weeks of storage at 40°C in a dry atmosphere, is 12.8% equating to 98% stability.

### Example 4:

The procedure of Example 1 was repeated with a different kind of oleoresin, and quite different amounts of other products.

The initial mixed carotenoids oleoresin is extracted from different sources: tomato, marigold, palm oil. It particularly contains at least 60% of β-carotene and 21 % of lutein and zeaxanthin.

| COMPONENT | CONCENTRATION of wet granules (%) | CONCENTRATION of dry granules (%) | WEIGHT (grams) |
|---|---|---|---|
| Gelatine 140 bloom | 15.0 | 33.6 | 180 |
| Lactose | 18.3 | 27.5 | 220 |
| Water | 50.0 | 8.0 | 600 |
| Mixed carotenoids oleoresine | 11.7 | 21.3 | 140 |
| Stearic acid | 5.00 | 9.1 | 60 |
| Silica | | 0.5 | |
| TOTAL | 100 | 100 | 1200 |

After formation of the droplets. Then, 40 g of an aqueous solution of 16.7% glutaraldehyde is added in the methyl ester rapeseed oil. After 20 minutes, the granules are filtrated, mixed with silica and dried one hour at room temperature and one hour at 60°C (air inlet temperature).

The particle size of the granules obtained ranged from 100 to 1000 microns with 35% in the range from 160 to 630 microns.

Because the initial oleoresin contains 60% of β-carotene and 21 % of lutein , the theoretical amount in the particulate composition was calculated to be 12.8 % of β-carotene and 4.5% of lutein. The amount of lutein and zeaxanthin in granules measured one week after manufacturing is 12.6 % of β-carotene and 4.4% of lutein and zeaxanthin.

### Example 5:

This example is relative to purified lutein.

The glucose syrup containing 30% of water is dissolved in warm water (60C°). The gelatine is added to the glucose solution, stirring at a speed of 2 metres per second with a high-shear impeller.

The crystals of purified lutein mixed with the antioxidant are dispersed in the aqueous phase with a ultra-turrax impeller (used at 6000 rpm). This dispersion is poured under stirring (helix impeller at 2.6 m/sec of peripherical speed) into 1.5 liter of methyl ester of rapeseed oil. Droplets of aqueous phase are obtained.

The droplets of aqueous phase are solidified by cooling the reactor at 20°C.

Then, 24 g of an aqueous solution of 25% glutaraldehyde are added in the rapeseed oil. After 20 minutes, the granules are filtrated, mixed with silica and dried one hour at room temperature and one hour at 60°C (air inlet temperature).

| COMPONENT | CONCENTRATION of wet granules (%) | CONCENTRATION of dry granules (%) | WEIGHT (grams) |
|---|---|---|---|
| Gelatine 140 bloom | 19.0 | 43.6 | 380 |
| Glucose syrup | 32.5 | 36.4 | 650 |
| Water | 42.5 | 8.0 | 850 |
| Purified cristallized lutein | 5.00 | 9.6 | 100 |
| Antioxidant (Rosemary extract) | 1.00 | 1.9 | 20 |
| Silica | | 0.5 | |
| TOTAL | 100 | 100 | 2000 |

The particle size of the granules obtained ranged from 200 to 800 microns.

The theoretical amount of lutein and zeaxanthin in the granules is 9.6 % and the amount of lutein and zeaxanthin in granules measured after manufacturing is 9.4% that gives a 98% yield.

The amount of lutein and zeaxanthin, determined after four weeks of storage at 40°C in a dry atmosphere, is 9% equating to 96% stability.

The stability was also determined by a sun test which consists to put a thin layer of granules under UV light (one hour of this light is equivalent to 9,6 hours of sun light). The amount of pigment (lutein and zeaxanthin) is measured after 2.5, 5, and 24 hours.

| Sun test | | | |
|---|---|---|---|
| Duration treatment | 2.5 h | 5 h | 24 h |
| Equivalent in day | 1 | 2.1 | 9.9 |
| % of residual pigment | 93% | 89% | 89% |

After a storage period of 9 months at 5°C, the amount of pigment remains as high as 8.6% which corresponds to 91 % of the initial yield. These data are very satisfactory.

### Example 6:

This example is also relative to purified lutein but the lutein and zeaxanthin amount is twice more important than in previous example.

The vegetable oil used in this case is still methyl ester of rapeseed oil.

| **COMPONENT COMPONENT** | **CONCENTRATION of wet granules (%)** | **CONCENTRATION of dry granules (%)** | **WEIGHT (grams)** |
|---|---|---|---|
| Gelatine 140 bloom | 20.0 | 37.4 | 400 |
| Glucose syrup | 25.0 | 32.8 | 500 |
| Water | 42.5 | 6.0 | 850 |
| Purified cristallized lutein | 10.5 | 19.6 | 210 |
| Antioxidant | 2.0 | 3.7 | 40 |
| Silica | | 0.5 | |
| TOTAL | 100 | 100 | 2000 |

The theoretical amount of lutein and zeaxanthin in the granules is 19.6 % and the amount of lutein and zeaxanthin in granules measured after manufacturing is 19% that gives a 97% yield.

The amount of lutein and zeaxanthin, determined after four weeks of storage at 40°C in a dry atmosphere, is 17.8% equating to 94% stability.

## Claims

1. Solid solvent-free granules having a size distribution of from 100 µm to 2000 µm comprising at least 5% (w/w) of carotenoid, gelatine and a sugar.

2. The granules of claim 1, wherein said carotenoid is selected from the group consisting of lutein, zeaxanthin and their mixtures, in their free form and/or their esterified form.

3. The granules of claim 2, wherein said carotenoid is an oleoresin comprising at least esterified lutein and esterified zeaxanthin.

4. The granules of claim 2, wherein said carotenoid is a saponified oleoresin comprising at least free lutein and free zeaxanthin.

5. The granules of claim 2, wherein said carotenoid is purified crystallized lutein.

6. The granules of anyone of claims 1 to 5, wherein the amount of carotenoid varies between 5 to 30% (w/w).

7. The granules of claim 6, wherein the amount of carotenoid varies from 10 to 20% (w/w).

8. The granules of claim 1, wherein the amount of gelatine varies from 15 to 50% (w/w).

9. The granules of claim 1, wherein the amount of sugar varies from 10 to 50% (w/w).

10. The granules of claim 1, further comprising at least a fatty material selected from the group consisting of fatty acids, fatty esters, derivatives thereof and waxes.

11. The granules of claim 1, further comprising at least one antioxidant.

12. The granules of claim 11, wherein said antioxidant is selected from the group consisting of rosemary extracts, wine polyphenols extracts, ascorbic acid, sodium ascorbate, ascorbyl palmitate, tocopherols, derivatives of tocopherols, vitamin C, 3-tertiary butyl-4-hydroxyanisole (BHA), 3,5-di-tertiary-4-hydroxytoluene (BHT), 6-ethoxy-1,2-dihydroxy-2,2,4-trimethylquinoline (ethoxyquine).

13. The granules of claim 11 or 12, wherein the antioxidant amounts up to 10% (w/w).

14. The granules of anyone of claims 1 to 13, comprising a finite amount of water less than 10% (w/w).

15. A process for producing solid granules as defined in anyone of claims 1 to 14, said process comprising the following steps:
(a) preparing an aqueous solution of at least gelatine and a sugar,
(b) adding at least said carotenoid in said aqueous solution,
(c) adding the preparation obtained in (b) to an oil to obtain an emulsion of granules,
(d) cooling said emulsion to solidify the granules, and
(e) recovering and drying the granules.

16. The process of claim 15, wherein said oil of step (c) is a vegetable oil selected from the group consisting of rapeseed oil, corn oil, sunflower oil, soybean oil, palm oil and any ester thereof.

17. The process of claim 15, wherein said carotenoid is selected from the group consisting of lutein, zeaxanthin and their mixtures, in their free form and/or their esterified form.

18. The process of claim 17, wherein said carotenoid is an oleoresin comprising at least esterified lutein and esterified zeaxanthin.

19. The process of claim 17, wherein said carotenoid is a saponified oleoresin comprising at least free lutein and free zeaxanthin.

20. The process of claim 18 or 19, wherein the oleoresin is obtained from Marigold flowers and tomato.

21. The process of claim 17, wherein said carotenoid is purified crystallized lutein.

22. The process of claim 17, wherein the amount of carotenoid added to the aqueous solution is such that the amount of said carotenoid in said granules is from 5 to 30% (w/w).

23. The process of claim 22, wherein said carotenoid amounts from 10 to 20% (w/w) in said granules.

24. The process of claim 15, wherein before adding the carotenoid to the aqueous solution, said carotenoid is mixed with a fatty material selected from the group consisting of fatty acids, fatty esters, derivatives thereof and waxes.

25. The process of claim 24, wherein said fatty material has a vegetable origin.

26. The process of claim 24 or 25, wherein said fatty acid is selected from the group consisting of stearic acid, and the mixtures of stearic acid and palmitic acid.

27. The process of claim 15, wherein the amount of gelatine in the in aqueous solution is such that the gelatine amounts from 15 to 50% (w/w) in said granules.

28. The process of claim 27, wherein the amount of gelatine in said granules is from 30 to 45% (w/w).

29. The process of claim 15, wherein the sugar is selected from the group consisting of polyols, monosaccharides, disaccharides, glucose syrups and maltodextrines.

30. The process of claim 29, wherein the polyols are selected from the group consisting of glycerol, sorbitol, maltitol and xylitol; the monosaccharides are selected from the group consisting of fructose and glucose; and the disaccharides are selected from the group consisting of lactose, maltose and sucrose.

31. The process of claim 15, wherein the amount of sugar in the aqueous solution is such that the sugar amounts from 10 to 50% (w/w) in the granules.

32. The process of claim 15, wherein the aqueous solution is prepared at a temperature from 50 to 70°C.

33. The process of claim 17, wherein before adding the carotenoid to the aqueous solution, said carotenoid is mixed with at least one antioxidant.

34. The process of claim 33, wherein said antioxidant is miscible with oil and is selected from the group consisting of rosemary extracts, wine polyphenols extracts, ascorbic acid, sodium ascorbate, ascorbyl palmitate, tocopherols, derivatives of tocopherols, vitamin C, 3-tertiary butyl-4-hydroxyanisole (BHA), 3,5-di-tertiary-4-hydroxytoluene (BHT), 6-ethoxy-1,2-dihydroxy-2,2,4-trimethylquinoline (ethoxyquine).

35. The process of claim 34, wherein the amount of said antioxidant is such that it amounts up to 10% (w/w) in said granules.

36. The process of claim 15, wherein at step (d), the emulsion is cooled at a temperature below the glass transition temperature of the gelatine.

37. The process of claim 15, comprising a further step of adding a gelatine-crosslinking agent before step (e).

38. Nutraceutical composition comprising granules as defined in anyone of claims 1 to 14.
